# EUROPEAN PATENT SPECIFICATION

(11) **EP 1 416 791 B1**
(45) Date of publication and mention of the grant of the patent: **24.05.2006**
(21) Application number: 02767366.4
(22) Date of filing: 12.08.2002
(51) Int. Cl.: A01K 67/027, C07K 14/475, C07K 14/495

(54) **AN ANIMAL MODEL EXHIBITING CANCER, PULMONARY EMPHYSEMA AND CARDIOMYOPATHY**
TIERMODELL MIT KREBS, LUNGENEMPHYSEM UND KARDIOMYOPATHIE
MODELE ANIMAL PRESENTANT UN CANCER, UN EMPHYSEME PULMONAIRE ET UNE CARDIOMYOPATHIE

(30) Priority: 14.08.2001 US 312164 P
(43) Date of publication of application: 12.05.2004
(73) Proprietor: FrankGen Biotechnologie AG, 61476 Kronberg/Taunus (DE)
(72) Inventor: MELCHNER, Harald von, 61476 Kronbert/Taunus (DE); THOREY, Irmgard,, 81245 München (DE); WEMPE, Frank, 63165 Mühlheim am Main (DE); STERNER-KOCK, Anja, Institut für Veterinärspatholo, 14163 Berlin (DE); KESKI-OJA, Jorma, FIN-00660 Helsinki (FI)
(74) Representative: Helbing, Jörg
(86) International application number: PCT/EP2002/009011
(87) International publication number: WO 2003/015505

(56) References cited:
- EP-A- 1 092 768
- VON MELCHNER HARALD ET AL: "Disruption of the gene encoding the latent transforming growth factor beta binding protein 4 (LTBP4) causes severe pulmonary emphysema, cardiomyopathy and colorectal cancer." PROCEEDINGS OF THE AMERICAN ASSOCIATION FOR CANCER RESEARCH ANNUAL, vol. 43, March 2002 (2002-03), page 342 XP001153301 93rd Annual Meeting of the American Association for Cancer Research;San Francisco, California, USA; April 06-10, 2002, March, 2002 ISSN: 0197-016X
- SAHARINEN JUHA ET AL: "Identification and characterization of a new latent transforming growth factor-beta-binding protein, LTBP-4." JOURNAL OF BIOLOGICAL CHEMISTRY, vol. 273, no. 29, 17 July 1998 (1998-07-17), pages 18459-18469, XP002246446 ISSN: 0021-9258
- SHIPLEY J MICHAEL ET AL: "Developmental expression of latent transforming growth factor beta binding protein 2 and its requirement early in mouse development." MOLECULAR AND CELLULAR BIOLOGY, vol. 20, no. 13, July 2000 (2000-07), pages 4879-4887, XP002246447 ISSN: 0270-7306
- OEKLUE R ET AL: "THE LATENT TRANSFORMING GROWTH FACTOR BETA BINDING PROTEIN (LTBP) FAMILY" BIOCHEMICAL JOURNAL, THE BIOCHEMICAL SOCIETY, LONDON, GB, vol. 352, no. 3, 15 October 2000 (2000-10-15), pages 601-610, XP009010129 ISSN: 0264-6021
- STERNER-KOCK ANJA ET AL: "Disruption of the gene encoding the latent transforming growth factor-beta binding protein 4 (LTBP-4) causes abnormal lung development, cardiomyopathy, and colorectal cancer." GENES & DEVELOPMENT, vol. 16, no. 17, 1 September 2002 (2002-09-01), pages 2264-2273, XP002246448 September 1, 2002 ISSN: 0890-9369

### SEQUENCE LISTING

<110> FrankGen Biotechnologie AG
<120> AN ANIMAL MODEL EXHIBITING CANCER, PULMONARY EMPHYSEMA AND CARDIOMYOPATHY
<130> 292-6 PCT
<150> US SN 60/312,164
   <151> 2001-08-14
<160> 5
<170> PatentIn Ver. 2.1
<210> 1
   <211> 5440
   <212> DNA
   <213> Mus musculus
<220>
   <223> Long splice variant of mouse LTBP-4 cDNA
<400> 1
   ttgcacggtg gagaaagtga gtcggcctct gtctgaggtc gggggtaggg ggcgggaccg 60
   gggccaggcc gatccggacc cactggacac actgccctct tggagccgga ctctgcaccc 120
   gacacgatgc gccggcctgg cctcggcggc ccctgccccc tcttgctgct gctgctgctc 180
   ccagccgcca catctgctag cggctccagt cccagcccca gccccagccc catcgagaaa 240
   gcggtggtcc cgagccacca ggccggtgtt gctgcctgtc actgctgcct agaccagaca 300
   cctaagagta gccgctgcac ccgagcttcc tgcagggtcc ggaactgccc acctgccaag 360
   tgcacaggtc tagaggggtg cctgacccca acaccttcgg tgcccagccc cagccgcagt 420
   cccgtggaga agagtcaggt atccctcaac tggcagccac tgactttgca ggaggcccga 480
   gctctgctga ggcaaaggcg gccccgaggg ccgtgggccc gggcattgct gaaaagaagg 540
   cccccacatc gcgcccctgc cggccaggcc cgggtcctat gtcccttgat ctgtcacaac 600
   ggtggtgtgt gtgtgaagcc tgatcgctgc ctttgccccc cggacttcgc tggcaaattc 660
   tgccaattgc attcttcggg ggcccggccc ccggccccag ccatgccggg ccttacccgc 720
   tccgtttata caatgccact agccaaccac cgcgatgatg aacacggagt agcatcgatg 780
   gtgagcgtgc acgtggagca ccctcaggag gcatcagtgg tggtgcacca agtggaacgt 840
   gtgtcgggcc cctgggagga ggcgaaccct gaagccctag ccagggctga agcggcggca 900
   cgggccgagg cggcggcgcc ctacacagtg ctggcacaga gcgcgccacg cgaggacggc 960
   tactcagacg cctccggctt cggttactgt tttagagaac tgcgcggaag cgaatgtgca 1020
   tcgccgctgc cagggctccg gacgcaggaa gtctgctgcc gaggagaggg cttggcctgg 1080
   ggtgttcatg attgtcaccc gtgcgcggaa cacctgagga actccaacca agtgagtggc 1140
   ccaaatggac cttgtccacc cggcttcgag agagttaatg ggtcctgtgt agatgtggat 1200
   gagtgcgcca ccggtgggcg ttgccaacac ggggagtgtg ccaacactcg tggcggctac 1260
   acgtgtgtat gtccggacgg tttcctcctg gactcttccc gcagcagctg catctcccaa 1320
   cacgtgatct ccgaggcaaa ggggccctgc taccgagtgc tgcatgatgg cggatgctcg 1380
   ctgcccattc ttcgaaatat caccaaacag atctgctgct gtagccgtgt gggcaaggcg 1440
   tggggccgag gctgccagct ctgtccacct tatggttcag agggttttcg ggagatctgt 1500
   ccagctggcc ctggctacca ctattcagct tctgacctcc gatacaacac cagaccccta 1560
   aaccaggatc cacctcgagt gaccttcaat cagccacgtg ttccaccagc cacccctcgg 1620
   ccacctacag gctttctgcc tactcgtcga cctgagcccc gccctgaccc tgggccacag 1680
   cctgaacccc ggcctcggcc tgaaccccgg cctcggcctg aatcccgacc tcggcctgaa 1740
   ccccggcctc ggcctgaacc ccggcctcag cctgaatccc agcctaggcc tgaatcccgg 1800
   cctcggcctg aatcccagcc ttggcctgaa tttcccctgc ccagcatccc tgcttggacc 1860
   ggtccagaga ttcctgaatc aggtccctct tccagcatgt gtcagcgaaa tccccaggtt 1920
   tgtggtcctg gacgctgcgt tcctaggcca agcggctaca cttgtgcgtg cgacccaggt 1980
   ttccggctcg gcccccagga cactcgctgc attgacatag atgaatgtcg ccgcgtccct 2040
   acaccctgtg ctcctgggcg ttgcgagaat acaccaggca gctttcgctg cgtgtgcggc 2100
   acgggcttcc aagcaggccc gcgggctaca gagtgcctgg atgtggacga gtgccgccgc 2160
   gtgccgccgc cgtgtgaccg cgggcgctgc gagaacacgc caggcagttt cctatgtgtg 2220
   tgtcccgccg ggtaccaggc agcaccgcac ggagccagct gccaggatgt ggatgaatgc 2280
   acccagagcc caggcctctg tggtcgcggg gtctgcgaaa acctgcccgg ctctttccgc 2340
   tgtgtttgcc cggctggctt ccggggctcg gcgtgtgaag aagatgtgga tgaatgtgcc 2400
   cagcagcctc cgccttgcgg gccaggccgc tgcgacaaca ccgcgggttc gttccactgt 2460
   gcctgcccag ctggcttccg atcccgagga ccaggagccc cctgccaaga tgtggatgaa 2520
   tgttcccgga gcccctcgcc ttgtgcctat ggccgctgtg agaacacgga aggcagcttc 2580
   aagtgtgtct gcccaacagg cttccaaccc aacgctgcgg gctccgagtg cgaggatgtg 2640
   gatgagtgtg agaaccgctt ggcgtgtcca gggcaggagt gtgtgaactc accgggctcc 2700
   ttccagtgcc gggcctgccc tgttgggcac cacctgcacc gtggcagatg tactgatgtg 2760
   gatgaatgca gttcgggcac cccttgtggt ctccatggcc agtgcacaaa tactaaaggc 2820
   tccttccact gcagctgctc gacaggttac cgggcgccat ctggtcaacc tgggccttgt 2880
   gcagacataa atgagtgtct ggaaggcgac ttctgcttcc cccacggaga gtgcctcaac 2940
   actgatggct cctttacctg tacctgtgcc cctggctacc ggcctggacc tcgtggagca 3000
   tcttgtctgg acgtggacga gtgcagcgaa gaggatcttt gccagagcgg catctgtacc 3060
   aacactgacg gctccttcga gtgcatctgt cctcctggac accgcgctgg ccctgacctt 3120
   gcctcctgcc ttgacattga cgagtgtcgt gaacggggac ctgccctgtg cgggtctcag 3180
   cgctgtgaaa attctcctgg ttcctaccgc tgtgtgcggg actgcgatcc aggttaccac 3240
   cccggccctg agggcacctg tgatgacatt gatgagtgcc gagaatacgg ctctgcgatt 3300
   tgtggtgccc agcgctgtga gaacaccccg ggctcctacc gctgcacacc agcctgcgac 3360
   cctggctatc agcctacacc agggggcggg tgccaagatg tggatgaatg ccggaaccgg 3420
   tctttctgcg gcgcccatgc tatgtgccag aatctgcctg ggtccttcca gtgcgtctgt 3480
   gaccaaggct acgaaggggc acgggacgga cgtcactgcg tggatgtgaa tgagtgtgag 3540
   acactacagg gcgtgtgtgg ctccgcgctg tgtgaaaatg ttgaaggctc cttcctgtgt 3600
   gtctgcccca atagccctga ggaatttgat cccatgactg gacgctgtgt tcccccacgg 3660
   gctcctgcag gcacgttccc tggctcccag ccccaagcac ctgccagccc cagtcttcca 3720
   gccaggccac cagcccctcc cccacctcga agacccagcc ctcccaggca gggtcccgtg 3780
   agcagtggac gtcgcgagtg ctactttgat acagccgctc cagatgcgtg tgacaatatc 3840
   ttggcccgaa acgtgacgtg gcaggagtgc tgctgtactg tgggagaagg ctggggcagc 3900
   ggctgccgca tccaacagtg cccgggcacc gagacagctg aataccagtc attgtgtcct 3960
   cacggccggg gctacctggt gcccagtgga gacctgagtg cccggagaga tgtggacgaa 4020
   tgccaactct tccaggacca ggtgtgcaag agcggagtgt gtgtgaacac ggccccaggc 4080
   tactcttgct attgcagcaa cgggttctac tatcatgcac acaggctgga gtgcgttgat 4140
   aatgacgaat gtgcggacga ggagcctgct tgtgaaggtg gccgctgcgt caacacggtg 4200
   ggctcttacc actgtacctg cgagccccca ctggtgctgg acggctcccg gcgacgctgc 4260
   gtctccaacg agagccagag cctggatgac aatctaggag tgtgctggca ggaagtggga 4320
   cctgatctcg tctgcagtcg cccacgacta gaccggcagg ccacctacac cgagtgctgc 4380
   tgtctctatg gtgaagcctg gggtatggac tgcgctctct gtcctgccca ggactcagat 4440
   gactttgagg ccctttgcaa tgtgctgcgt ccacctgcct atggcccgcc gcgccctggt 4500
   ggctttggaa ttccctatga atatggccca gatataggcc caccttatca gagtctcccc 4560
   tatgggccag atctgtatcc accgccagtg ctaccctacg acccctaccc acccccacct 4620
   ggaccctttg ctcgccggga ggccccttat ggcgccccgc ccttcgacat gccggacttt 4680
   gaggatgatg gtggccccta tggcgagtct gagactcctg acccacctag ccgaggcact 4740
   ggttggcctt atcggtccag agacacccgt ggctccttcc cagagcctga ggaatcctct 4800
   gagcgtggaa gctatacagg agccttgtct gagccctatg agggcctaga ggccgaagag 4860
   tgtggtatcc tggatggctg ccctcacggc cgctgtgtac gcgttccaga aggcttcacc 4920
   tgcgattgtt tcgatggcta ccgcctggac atcacccgca tgtcctgtgt cgacgtcaac 4980
   gagtgtgatg aggcagaggc aacctcccca ctctgcatca atgcgcgctg tgtcaacact 5040
   gacggctcct tccgctgtat ctgtcgtcca ggatttgctc ccacgcatca gccacatcac 5100
   tgtgcgcctg ctcgaccccg ggcctgagca ggggtgccaa atctcccact atacctgagc 5160
   accgggagct tccagagcac gcctgcctcc ctccctctag tctgcatgtg tgcaaggatg 5220
   tcagacagtc tcgacttagg atggacagac acagaggggt gccttctatg gtgccagcct 5280
   gtcatgccca gtccctccca tcagtgccct gggcctgtcc tgggtcccct gacacattac 5340
   ttgccctttt atacaatttt caattaaaac acctattttg taaaaaaaaa aaaaaaaaaa 5400
   aaaaaaaaaa aaaaaaaaaa aaaaaaaaaa aaaaaaaaaa                       5440
<210> 2
   <211> 5120
   <212> DNA
   <213> Mus musculus
<220>
   <223> Short splice variant of mouse LTBP4 cDNA
<400> 2
   tggagcgcgg cgctgcagcc atggcgggcg gcgcgcagct gctctgggtg tcgctattgg 60
   tgctgctggc gcagctgggg ccgcagcccg tactgggccg accccgagag cgtcttcgcg 120
   tgcgcttcac ccccgctgtg tgcggcctgc gttgcatcca cgggcccact ggctcccgct 180
   gtaccccgac ctgcgcgccc cgcaacgcca ccagcgtgga cagcggcgcg cccggcgggg 240
   cggccccggg gggacccggc ttccgcgcct tcctatgtcc cttgatctgt cacaacggtg 300
   gtgtgtgtgt gaagcctgat cgctgccttt gccccccgga cttcgctggc aaattctgcc 360
   aattgcattc ttcgggggcc cggcccccgg ccccagccat gccgggcctt acccgctccg 420
   tttatacaat gccactagcc aaccaccgcg atgatgaaca cggagtagca tcgatggtga 480
   gcgtgcacgt ggagcaccct caggaggcat cagtggtggt gcaccaagtg gaacgtgtgt 540
   cgggcccctg ggaggaggcg aaccctgaag ccctagccag ggctgaagcg gcggcacggg 600
   ccgaggcggc ggcgccctac acagtgctgg cacagagcgc gccacgcgag gacggctact 660
   cagacgcctc cggcttcggt tactgtttta gagaactgcg cggaagcgaa tgtgcatcgc 720
   cgctgccagg gctccggacg caggaagtct gctgccgagg agagggcttg gcctggggtg 780
   ttcatgattg tcacccgtgc gcggaacacc tgaggaactc caaccaagtg agtggcccaa 840
   atggaccttg tccacccggc ttcgagagag ttaatgggtc ctgtgtagat gtggatgagt 900
   gcgccaccgg tgggcgttgc caacacgggg agtgtgccaa cactcgtggc ggctacacgt 960
   gtgtatgtcc ggacggtttc ctcctggact cttcccgcag cagctgcatc tcccaacacg 1020
   tgatctccga ggcaaagggg ccctgctacc gagtgctgca tgatggcgga tgctcgctgc 1080
   ccattcttcg aaatatcacc aaacagatct gctgctgtag ccgtgtgggc aaggcgtggg 1140
   gccgaggctg ccagctctgt ccaccttatg gttcagaggg ttttcgggag atctgtccag 1200
   ctggccctgg ctaccactat tcagcttctg acctccgata caacaccaga cccctaaacc 1260
   aggatccacc tcgagtgacc ttcaatcagc cacgtgttcc accagccacc cctcggccac 1320
   ctacaggctt tctgcctact cgtcgacctg agccccgccc tgaccctggg ccacagcctg 1380
   aaccccggcc tcggcctgaa ccccggcctc ggcctgaatc ccgacctcgg cctgaacccc 1440
   ggcctcggcc tgaaccccgg cctcagcctg aatcccagcc taggcctgaa tcccggcctc 1500
   ggcctgaatc ccagccttgg cctgaatttc ccctgcccag catccctgct tggaccggtc 1560
   cagagattcc tgaatcaggt ccctcttcca gcatgtgtca gcgaaatccc caggtttgtg 1620
   gtcctggacg ctgcgttcct aggccaagcg gctacacttg tgcgtgcgac ccaggtttcc 1680
   ggctcggccc ccagggcact cgctgcattg acatagatga atgtcgccgc gtccctacac 1740
   cctgtgctcc tgggcgttgc gagaatacac caggcagctt tcgctgcgtg tgcggcacgg 1800
   gcttccaagc aggcccgcgg gctacagagt gcctggatgt ggacgagtgc cgccgcgtgc 1860
   cgccgccgtg tgaccgcggg cgctgcgaga acacgccagg cagtttccta tgtgtgtgtc 1920
   ccgccgggta ccaggcagca ccgcacggag ccagctgcca ggatgtggat gaatgcaccc 1980
   agagcccagg cctctgtggt cgcggggtct gcgaaaacct gcccggctct ttccgctgtg 2040
   tttgcccggc tggcttccgg ggctcggcgt gtgaagaaga tgtggatgaa tgtgcccagc 2100
   agcctccgcc ttgcgggcca ggccgctgcg acaacaccgc gggttcgttc cactgtgcct 2160
   gcccagctgg cttccgatcc cgaggaccag gagccccctg ccaagatgtg gatgaatgtt 2220
   cccggagccc ctcgccttgt gcctatggcc gctgtgagaa cacggaaggc agcttcaagt 2280
   gtgtctgccc aacaggcttc caacccaacg ctgcgggctc cgagtgcgag gatgtggatg 2340
   agtgtgagaa ccgcttggcg tgtccagggc aggagtgtgt gaactcaccg ggctccttcc 2400
   agtgccgggc ctgccctgtt gggcaccacc tgcaccgtgg cagatgtact gatgtggatg 2460
   aatgcagttc gggcacccct tgtggtctcc atggccagtg cacaaatact aaaggctcct 2520
   tccactgcag ctgctcgaca ggttaccggg cgccatctgg tcaacctggg ccttgtgcag 2580
   acataaatga gtgtctggaa ggcgacttct gcttccccca cggagagtgc ctcaacactg 2640
   atggctcctt tacctgtacc tgtgcccctg gctaccggcc tggacctcgt ggagcatctt 2700
   gtctggacgt ggacgagtgc agcgaagagg atctttgcca gagcggcatc tgtaccaaca 2760
   ctgacggctc cttcgagtgc atctgtcctc ctggacaccg cgctggccct gaccttgcct 2820
   cctgccttga cattgacgag tgtcgtgaac ggggacctgc cctgtgcggg tctcagcgct 2880
   gtgaaaattc tcctggttcc taccgctgtg tgcgggactg cgatccaggt taccaccccg 2940
   gccctgaggg cacctgtgat gacattgatg agtgccgaga atacggctct gcgatttgtg 3000
   gtgcccagcg ctgtgagaac accccgggct cctaccgctg cacaccagcc tgcgaccctg 3060
   gctatcagcc tacaccaggg ggcgggtgcc aagatgtgga tgaatgccgg aaccggtctt 3120
   tctgcggcgc ccatgctatg tgccagaatc tgcctgggtc cttccagtgc gtctgtgacc 3180
   aaggctacga aggggcacgg gacggacgtc actgcgtgga tgtgaatgag tgtgagacac 3240
   tacagggcgt gtgtggctcc gcgctgtgtg aaaatgttga aggctccttc ctgtgtgtct 3300
   gccccaatag ccctgaggaa tttgatccca tgactggacg ctgtgttccc ccacgggctc 3360
   ctgcaggcac gttccctggc tcccagcccc aagcacctgc cagccccagt cttccagcca 3420
   ggccaccagc ccctccccca cctcgaagac ccagccctcc caggcagggt cccgtgagca 3480
   gtggacgtcg cgagtgctac tttgatacag ccgctccaga tgcgtgtgac aatatcttgg 3540
   cccgaaacgt gacgtggcag gagtgctgct gtactgtggg agaaggctgg ggcagcggct 3600
   gccgcatcca acagtgcccg ggcaccgaga cagctgaata ccagtcattg tgtcctcacg 3660
   gccggggcta cctggtgccc agtggagacc tgagtgcccg gagagatgtg gacgaatgcc 3720
   aactcttcca ggaccaggtg tgcaagagcg gagtgtgtgt gaacacggcc ccaggctact 3780
   cttgctattg cagcaacggg ttctactatc atgcacacag gctggagtgc gttgataatg 3840
   acgaatgtgc ggacgaggag cctgcttgtg aaggtggccg ctgcgtcaac acggtgggct 3900
   cttaccactg tacctgcgag cccccactgg tgctggacgg ctcccggcga cgctgcgtct 3960
   ccaacgagag ccagagcctg gatgacaatc taggagtgtg ctggcaggaa gtgggacctg 4020
   atctcgtctg cagtcgccca cgactagacc ggcaggccac ctacaccgag tgctgctgtc 4080
   tctatggtga agcctggggt atggactgcg ctctctgtcc tgcccaggac tcagatgact 4140
   ttgaggccct ttgcaatgtg ctgcgtccac ctgcctatgg cccgccgcgc cctggtggct 4200
   ttggaattcc ctatgaatat ggcccagata taggcccacc ttatcagagt ctcccctatg 4260
   ggccagatct gtatccaccg ccagtgctac cctacgaccc ctacccaccc ccacctggac 4320
   cctttgctcg ccgggaggcc ccttatggcg ccccgccctt cgacatgccg gactttgagg 4380
   atgatggtgg cccctatggc gagtctgaga ctcctgaccc acctagccga ggcactggtt 4440
   ggccttatcg gtccagagac acccgtggct ccttcccaga gcctgaggaa tcctctgagc 4500
   gtggaagcta tacaggagcc ttgtctgagc cctatgaggg cctagaggcc gaagagtgtg 4560
   gtatcctgga tggctgccct cacggccgct gtgtacgcgt tccagaaggc ttcacctgcg 4620
   attgtttcga tggctaccgc ctggacatca cccgcatgtc ctgtgtcgac gtcaacgagt 4680
   gtgatgaggc agaggcaacc tccccactct gcatcaatgc gcgctgtgtc aacactgacg 4740
   gctccttccg ctgtatctgt cgtccaggat ttgctcccac gcatcagcca catcactgtg 4800
   cgcctgctcg accccgggcc tgagcagggg tgccaaatct cccactatac ctgagcaccg 4860
   ggagcttcca gagcacgcct gcctccctcc ctctagtctg catgtgtgca aggatgtcag 4920
   acagtctcga cttaggatgg acagacacag aggggtgcct tctatggtgc cagcctgtca 4980
   tgcccagtcc ctcccatcag tgccctgggc ctgtcctggg tcccctgaca cattacttgc 5040
   ccttttatac aattttcaat taaaacacct attttgtaaa aaaaaaaaaa aaaaaaaaaa 5100
   aaaaaaaaaa aaaaaaaaaa 5120
<210> 3
   <211> 24
   <212> DNA
   <213> Artificial Sequence
<220>
   <223> Description of Artificial Sequence: Primer
<400> 3
   aagagggaga agaaactggg ctgg 24
<210> 4
   <211> 20
   <212> DNA
   <213> Artificial Sequence
<220>
   <223> Description of Artificial Sequence: Primer
<400> 4
   tcctgtagtg tttgcccctg 20
<210> 5
   <211> 3036
   <212> DNA
   <213> Artificial Sequence
<220>
   <223> Intron 5 of mouse LTBP4 gene
<220>
   <223> Description of Artificial Sequence: Primer
<400> 5

## Description

The present invention relates to a non-human animal model and cells which do not produce functional latent transforming growth factor β binding protein 4 or which produce suboptimal levels of latent transforming growth factor β binding protein 4. Furthermore, the present invention relates to methods for ex-vivo diagnosing cancer, pulmonary emphysema or cardiomyopathy and analyzing whether cancer and/or pulmonary emphysema and/or cardiomyopathy are caused by a differential expression of LTBP-4 or by a defect in LTBP-4 gene. Moreover the present invention relates to selecting an agent for treating a symptom occurring in the animal model of the invention.

### Background of the Invention

Transforming growth factor - β (TGF- β) belongs to a protein superfamily whose members are necessary for normal development. They also control cell growth and differentiation in a variety of adult tissues and are involved in a wide range of immune reactions (reviewed in Massague, J., et al. (2000) *Cell* 103, 295-309, Massague, J. & Wotton, D. (2000) *Embo J* 19, 1745-1754). Most cells secrete TGF-β as a functionally inactive (latent) precursor cytokine that interacts with its receptors only after proteolytic activation. There are two known, structurally different latent complexes of TGF- β. A small latent complex consists of a mature TGF- β dimer bound to the N-terminal portion of the TGF- β precursor, i.e. the latency associated peptide (LAP). The large latent complex contains, in addition to TGF- β -LAP, the latent TGF- β binding protein (LTBP). Unless proteolytically cleaved from LTBPs after secretion, TGF- β is deposited in the extracellular matrix (ECM) as a high molecular weight complex with LTBP and LAP (reviewed in Koli, K, et al. (2001) *Microsc Res Tech* 52, 354-362., Taipale, J., et al. (1998) *Adv Cancer Res* 75, 87-134).

LTBPs are glycoproteins that belong to the family of fibrillins. More than 60% of the protein structure of the LTBP family is composed of repetitive EGF like domains and 8-Cystein repeats, both of which are believed to mediate protein-protein interactions. Like fibrillins, LTBPs are structural components of the extracellular matrix (ECM) and bind to elastic fibers. Although LTBPs are not directly involved in TGF- β latency most cells secrete TGF- β in complex with LTBP and, by binding TGF- β to the ECM, form extracellular deposits of TGF- β. This is believed to provide tissues with a rapidly inducible and highly localized TGF- β signal (Koli, K. et al. (2001) *Microsc Res Tech* 52, 354-362). Four isoforms of LTBPs (LTBP 1-4) have been cloned thus far (Tsuji, T. et al. (1990) *Proc Natl Acad Sci U S A* 87, 8835-8839, Moren, A. et al. (1994) *J Biol Chem* 269, 32469-32478, Yin, W. et al. (1995) *J Biol Chem* 270, 10147-10160, Giltay, R., et al. (1997) *FEBS Lett* 411, 164-168, Saharinen, J. et al. (1998) *J Biol Chem* 273, 18459-18469). Each has several splice variants and at least one (LTBP1) has two alternative promoters (Koski, C., et al. (1999) *J Biol Chem* 274, 32619-32630). The expression of both, isoforms and splice variants, appears tightly controlled in a tissue specific manner. The function of LTBPs at organismal level is largely unknown (Shipley, J. et al. (2000) Mol. Cell. Biol., 4879-4887, Öklü, R. et al. (2000) Biochem. J., 601-610).

Furthermore, EP-A-1 092768 describes gene trapping methods.

As it is evident from the prior art there is a need for animal models to elucidate the function of LTBPs and in particular LTBP-4. A further object is to provide cells lacking LTBP-4 protein.

As a solution to this object a non-human animal model and cells which do not produce functional latent transforming growth factor β binding protein 4 (LTBP-4) or which produce suboptimal levels of latent transforming growth factor β binding protein 4 (LTBP-4) were provided.

### Description of the Invention

The present invention relates to a non-human animal model which does not produce functional latent transforming growth factor β binding protein 4 (LTBP-4) or which produces suboptimal levels of latent transforming growth factor factor β binding protein 4 (LTBP-4), wherein the animal model exhibits cancer (e.g. a colon cancer), pulmonary emphysema or cardiomyopathy.

In a preferred embodiment the genome of the animal comprises a homozygous disruption of the LTBP-4 gene. Preferably this homozygous disruption has been generated by a mutation and this mutation can be an insertion, deletion or a substitution mutation. Furthermore, preferably said mutation is generated by gene targeting, gene trap integration or mutagenesis and it has occurred in an exon, intron, regulatory region or splicing site of the LTBP-4 gene, preferably in intron 5 of the LTBP-4 gene.

Furthermore, the animal is preferably a mammal, more preferably a mouse or a rat.

In a further aspect the invention relates to a cell isolated from a non-human animal model whose genome comprises a homozygous disruption of the LTBP-4 gene such that said gene does not produce functional latent transforming growth factor β binding protein 4 or such that said gene produces suboptimal levels of latent transforming growth factor β binding protein 4. Preferably, the cell is from a colon, lung or heart.

In a further aspect the invention is directed to a non-human cell whose genome comprises a homozygous disruption of the LTBP-4 gene. The cell is, preferably, an embryonic stem cell.

In a further embodiment the present invention is directed to a method for selecting an agent for treating a symptom occurring in the animal model of the invention comprising: a) applying one or more agents to be tested to the animal model of the invention; and b) determining whether one or more symptoms occurring in the animal model of the present invention have changed as a result of application of said agent or agents. In a preferred embodiment the symptom is selected from a group consisting of cancer, pulmonary emphysema and cardiomyopathy.

The agent suitable for treating a symptom occurring in the animal model of the invention may be used as a pharmaceutical. Furthermore, the agent which is suitable for treating a symptom occurring in the animal model of the invention may be used for the preparation of a pharmaceutical composition for the treatment of cancer, pulmonary emphysema or cardiomyopathy. Moreover, treatment of cancer, pulmonary emphysema or cardiomyopathy using the agent which is suitable for treating a symptom occurring in the animal model of the present invention may be possible.

A further aspect of the invention is a method to analyze whether cancer, pulmonary emphysema or cardiomyopathy is caused by differential LTBP-4 gene or protein expression or expression level or by a defect in the LTBP-4 gene comprising: a) characterizing the LTBP-4 gene or protein expression or expression level or LTBP-4 gene allele status of an individual having cancer and/or pulmonary emphysema and/or cardiomyopathy, and b) characterizing the LTBP-4 gene or protein expression or expression level or LTBP-4 gene allele status of a control, wherein the method is not practised on the human or animal body and wherein a difference in the LTBP-4 gene or protein expression or expression level or LTBP-4 gene allele status indicates that cancer and/or pulmonary emphysema and/or cardiomyopathy is caused by differential LTBP-4 gene or protein expression or expression level or by a defect in the LTBP-4 gene.

Furthermore, one aspect of the invention relates to a method for diagnosing cancer, pulmonary emphysema or cardiomyopathy comprising: a) characterizing the LTBP-4 gene or protein expression or expression level or LTBP-4 gene allele status of an individual, b) characterizing the LTBP-4 gene or protein expression or expression level or LTBP-4 gene allele status of a control, wherein the method is not practised on the human or animal body and wherein a difference in the LTBP-4 gene or protein expression or expression level or LTBP-4 gene allele status indicates the presence of cancer and/or pulmonary emphysema and/or cardiomyopathy in said individual.

In a preferred embodiment of the methods to analyze whether cancer and/or pulmonary emphysema and/or cardiomyopathy is caused by LTBP-4 and for diagnosing cancer, pulmonary emphysema or cardiomyopathy, the LTBP-4 gene and the LTBP-4 expression or expression level is detected by RT-PCR, Northern analysis, microarray (DNA chip) analysis or antibodies directed to LTBP-4 protein and the LTBP-4 gene allele status is detected by mutation screening.

The term "does not produce functional latent transforming growth factor β binding protein 4 (LTBP-4)" denotes the lack of translation of an effective LTBP-4 protein.

The term "produces suboptimal levels of latent transforming growth factor β binding protein 4 (LTBP-4)" encompasses the translation level of LTBP-4 protein; which is insufficient to produce an effective concentration of LTBP-4. Preferably, the level is reduced at least by 50%, more preferably by 70% and most preferably by 95 %.

The term "homozygous disruption of the LTBP-4 gene" relates to an identical mutation in both LTBP-4 alleles.

The term "mutation" refers to a change of one or more nucleotide pairs of a DNA molecule.

The term "insertion" is directed to a mutation identified by the presence of one or more additional base pairs in the DNA. The term "deletion" relates to a mutation generated by removal of a sequence of DNA (one or more base pairs), the regions on either side being joined together. The term "substitution mutation" is directed to a nucleotide change. The substitution mutation can result in an amino acid change or can introduce a premature translation stop codon. Furthermore, a substitution mutation can affect splicing or expression of the gene when occurring in the sites necessary for splicing or regulatory regions of the gene.

The term "gene targeting" relates to a type of homologous recombination that occurs when a fragment of genomic DNA is introduced into a cell and that fragment locates and recombinates with homologous sequences. The term "gene trap integration" is directed to insertion of a vector, which comprises a reporter gene and which is activated upon its insertion within an active transcription unit, into a chromosomal site. The term "mutagenesis" denotes a chemical or physical treatment that changes nucleotides in the genome of an organism. An example of a chemical mutagenesis is N-ethyl-N-nitrosurea (ENU) mutagenesis.

The term "exon" encompasses a segment of a gene which is decoded to give a mRNA product or a mature RNA product. Individual exons may contain protein coding DNA and/or noncoding DNA. The term "intron" denotes noncoding DNA which separates neighboring exons in a gene. During gene expression introns, like exons are transcribed into RNA but the transcribed intron sequences are subsequently removed by RNA splicing and are not present in mRNA. The term "regulatory region" relates to the nucleotide sequence which comprises regions which are necessary for the regulation of the transcription of the gene. These regions comprise e.g. promoters and enhancers and they can be located in 5' region of the exons or in introns. The term "splicing site" encompasses the nucleotides at a) the end of an exon and the beginning of the intron and at b) the end of the intron and beginning of the exon that are required for the joining of two exons and removal of an intron during the processing of a primary transcript to a functional mRNA.

The term "cancer" refers to a neoplastic disease. Cancer cells unlike benign tumor cells exhibit the properties of invasion and metastasis and are highly dedifferentiated. The term "pulmonary emphysema" denotes a condition of the lung characterized by a size increase beyond normal of air spaces distal to the terminal bronchioles, caused either by dilatation of alveoli or by destruction of their walls. The term "cardiomyopathy" designates a primary noninflammatory disease of the heart muscle which is not a result of ischemic, hypertensive, congenital, valvular or percardial disease.

The term "embryonic stem cell" denotes a cell line derived from undifferentiated, totipotent cells from a non-hunan embryo.

The term "selecting an agent for treating a symptom " encompasses choosing a composition for management of the condition.

The term "symptom" relates to subjective evidence of disease in an individual's condition.

The term "applying one or more agents" relates to administering one or more compositions. The compositions can be administered orally, by inhalation, parenterally, e.g. intravenously, subcutaneously, intraperitoneally or intramuscularly, or topically, e.g. ophtalmically, vaginally, rectally or intranasally.

The term "differential LTBP-4 gene or protein expression or expression level" denotes transcription or translation of LTBP-4 gene, which is reduced or increased. Preferably, there is a lack of transcription or translation.

The term "defect in the LTBP-4 gene" relates to a mutation, i.e. a nucleotide change in the LTBP-4 gene.

The term "LTBP-4 gene allele status" denotes the state of the DNA sequence in both alleles of LTBP-4, i.e. whether the LTBP-4 gene contains a mutation and whether this mutation is heterozygous or homozygous.

The term "RT-PCR" encompasses a two-step protocol for synthesizing cDNA molecules, wherein cDNA strands are synthesized by reverse transcriptase, mRNA as a template, and the specific cDNA strand is amplified by PCR. The term "Northern analysis" relates to a molecular hybridization method in which the target consists of RNA molecules that have been size fractionated by gel electrophoresis and subsequently transferred to a membrane. The term "microarray analysis" refers to large scale expression analyses where tens of thousands of mRNAs from an individual are immobilized on a solid suface, preferably glass, and are hybridized to a LTBP-4 specific probe. The term "antibody directed to LTBP-4 protein" denotes a protein that is synthesized by a B lymphocyte and recognizes specific epitopes on a LTBP-4 protein.

The term "mutation screening" relates to searching for nucleotide changes in the DNA or cDNA of an individual. This can be carried out e.g. by sequencing.

The term "means to detect the LTBP-4 gene or protein expression or expression level or LTBP-4 gene allele status" denotes resources to characterize whether LTBP-4 gene is transcribed or LTBP-4 protein is translated and whether the level of transcription/translation is normal, decreased or increased or whether LTBP-4 gene carries a nucleotide change. The resources comprise antibodies directed to LTBP-4 protein, a probe, which hybridizes to LTBP-4 gene and primer pairs which are suitable to amplify regions of LTBP4-gene or LTBP-4 cDNA.

In the present invention a strong hypomorphic allele of the mouse LTBP-4 gene was generated by gene trap mutagenesis in embryonic stem cells. Mice homozygous for the gene trap integration expressed only trace amounts of LTBP-4 and developed severe pulmonary emphysema, cardiomyopathy and colorectal cancer. The abnormalities were caused by a defect in the assembly of elastic fibers and by a defective TGF- β transport to the extracellular space.

Interestingly, the hypomorphic allele was generated by an inverse orientation exon trap insertion into an intron of the LTBP-4 gene, which theoretically should not interfere with gene expression. However, intron insertions have been shown previously to cause mutations. Examples include the ob2J mutation induced by a retrotransposon insertion into the first intron of the mouse leptin gene (Moon, B.C. & Friedman, J.M. (1997) *Genomics* 42, 152-156), the mdr-3 mutation induced by a retroviral LTR insertion into the 22^{nd} intron of the mouse multi-drug resistance gene -3 (Jun, K., et al. (2000) *Mamm Genome* 11, 843-848), and the Mbp mutation induced by a retrotransposon insertion into the 3^{rd} intron of the rat myelin basic protein gene (O'Connor, L.T. et al. (1999) *J Neurosci* 19, 3404-3413). In each case, aberrant splicing precluded the expression of a fully processed, full length transcript. In cells of the present invention multiple atypical LTBP-4 transcripts were detected by RT-PCR, indicating that the mutation was caused by aberrant splicing.

The disease model of the present invention demonstrates a dual function of the TGF- β binding proteins. While the absence of elastic fibers in the lung and colon underscores the structural requirement of LTBP-4, the lack of extracellular TGF- β implicates LTBP-4 in TGF- β signaling. As TGF- β inhibits epithelial cell proliferation, particularly in the colon, it can be concluded that its absence from the colonic ECM is the most likely oncogenic trigger for the development of colon cancer in mice of the present invention. Indeed, several studies have associated defects in TGF-β signaling with colorectal cancer. For example, mice with null mutations in the TGF-β signal transducing protein -Smad 3, develop tumors which are similar to the tumors growing in 3C7 mice (Zhu, Y., et al. (1998) *Cell* 94, 703-714). Furthermore, mutations in the TGF- β signal transducing proteins Smad 2 and Smad 4 or mutations in the TGF- β II receptors are very common in human colorectal cancers, suggesting that TGF- β and its downstream targets have tumor suppressor functions (Markowitz, S. *et al*. (1995) *Science* 268, 1336-1338), Riggins, G.J. et al. (1997) *Cancer Res* 57, 2578-2580, Zhou, S. *et al*. (1998) *Proc Natl Acad Sci U S A* 95, 2412-2416). Thus, the present invention demonstrates that LTBP-4 is important for the integrity of the ECM and prevents oncogenic transformation, cancer cell invasion and metastatic spread.

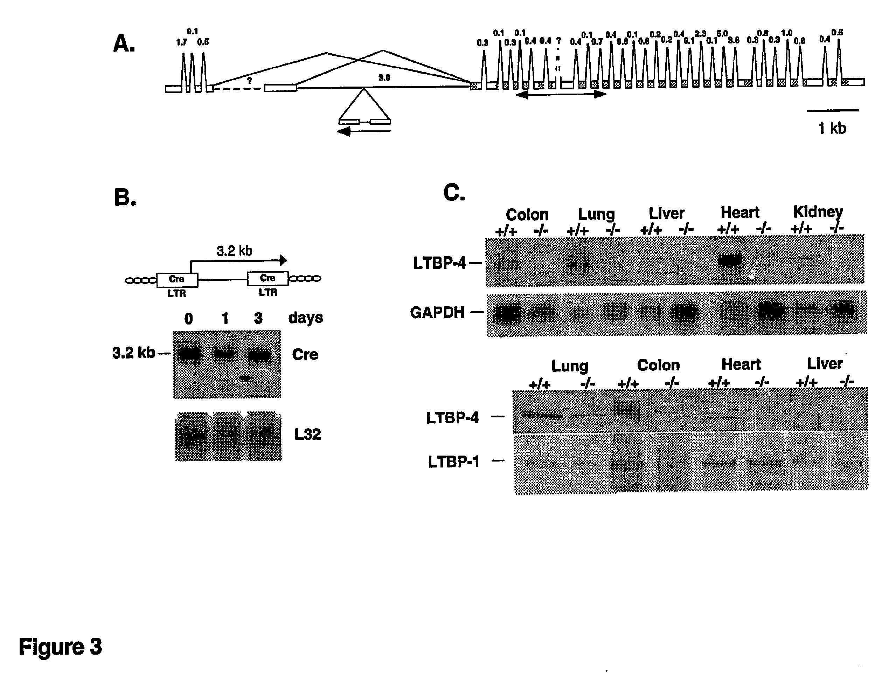

One of the most advantageous observations made in this invention is the tissue selectivity of the LTBP-4 mutation. Only the lung, heart and colon developed disease in mutant mice of the invention, indicating that LTBP-4 is a highly tissue specific protein. Moreover, this tissue specificity also shows that despite the extensive structural similarities, latent TGF-β binding proteins have little if any overlapping functions. Accordingly, the mutant mice express normal levels of LTBP-1 and LTBP-2, demonstrating that these isoforms cannot substitute for LTBP-4 (Figure 3C). In addition, gene knock out studies have assigned LTBP-2 and LTBP-3 to completely different functions. For example, mice expressing null alleles of LTBP-2 die very early in development, most likely due to defective implantation (Shipley, J.M. et al. (2000) *Mol Cell Biol* 20, 4879-4887). In contrast, LTBP-3 knock out mice develop extensive skeletal abnormalities due to defective bone formation.

In conclusion, the present invention provides an non human animal model of human disease that reveals some crucial functions of LTBP-4. In view of these functions, the animal model of the invention can be used to provide novel treatments for connective tissue disorders, pulmonary emphysema, cardiomyopathy and cancer, and to obtain information about cancer and about the normal and defected function of connective tissue, lung and heart. Furthermore, the animal model of the present invention can be used for the dissection of the molecular mechanisms of LTBP-4 and the TGF- β pathway and for the identification and cloning of modifier genes able to modify, aggravate, reduce or inhibit the phenotype associated with cancer, pulmonary emphysema, cardiomyopathy or other conditions occurring in the animal model of the invention. Moreover, the animal model can be used for identification of early diagnostic markers for cancer and/or pulmonary emphysema and/or cardiomyopathy or other conditions occurring in the animal model of the invention. In addition, the animal model of the present invention can be used for the monitoring of the activity of agents useful in the prevention or treatment of cancer and/or pulmonary emphysema and/or cardiomyopathy or other conditions occurring in the animal model of the invention and as a test model system for agents suspected of promoting or aggravating cancer and/or pulmonary emphysema and/or cardiomyopathy or other conditions occurring in the animal model of the invention.

### Description of Figures

The present invention is further illustrated by the following figures:

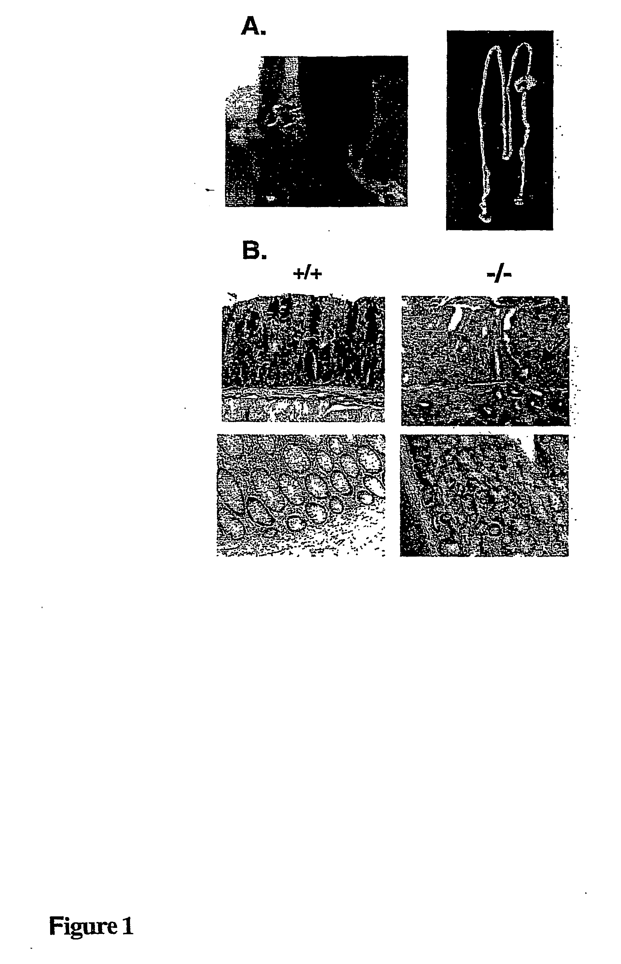

**Figure 1**: Colorectal cancer in mice of the present invention (the mice of the present invention designated herein as 3C7 mice). **A.** Rectal prolapse (left panel) and digestive tract preparation with colorectal tumor (arrow) (right panel) of a 4 month old mouse. **B**. Histological sections through the colorectal region of wild type (+/+) and homozygous 3C7 mice (-/-). Top: PAS staining at x 200 magnification. *Bottom:* Immunostaining for the proliferation associated antigen Ki-67. Arrow points to an aberrant, dysplastic crypt. The -/- phenotype was stable for over 6 generations of backcrossings to a C57BU6 genetic background.
**Figure 2:** Pulmonary emphysema and cardiomyopathy in 3C7 mice. **A**. Heart/lung preparations from wild type (+/+) and homozygous 3C7 (-/-) mice. **B.** Lungs. Hematoxylin/eosin stain at x 200 magnification. **C.** Hearts. Hematoxylinleosin stain at x 200 magnification. The -/- phenotype was stable for over 6 generations of backcrossings to a C57BU6 genetic background.
**Figure 3:** Gene trap disruption of the LTBP-4 gene locus. **A.** Predicted intron/exon structure of the mouse LTBP-4 gene with the gene trap integration site. The exon/intron structure was compiled by using the full length mouse cDNA sequence (SEQ ID NO:1, SEQ ID NO:2), the full length human cDNA sequence, and the available genomic sequence of mouse chromosome 7 synthenic to human chromosome 11. **B**. Structure of the U3Cre gene trap provirus *(top)* and Northern blot analysis of 3C7 ES cells (*bottom*). Polyadenylated mRNAs from differentiating ES cells were blotted onto nylon filters and hybridized to ³²P labeled Cre- or L32-specific probes. **C**. LTBP-4 expression in wild type (+/+) and homozygous 3C7 (-/-) mice. Top: Northern blot analysis. Polyadenylated RNAs from selected mouse tissues were blotted onto nylon filters and hybridized to a ³²P labeled LTBP-4 probe. The probe was a 505 kb fragment extending between nucleotides 862 -1367 of the LTBPS-4 cDNA (SEQ ID NO:2) (for location see A.). *Bottom:* immunoblotting with human anti-LTBP-4 and anti-LTBP-1 antibodies. Proteins from selected tissue digests were separated on denaturing polyacrylamide gels, transferred to nitrocellulose membranes and reacted with polyclonal anti-LTBP-1 or anti-LTBP-4 antibodies.
**Figure 4:** Sequence of intron 5 of the mouse LTBP-4 gene. An arrow indicates the gene trap integration site. Splice donor and splice acceptor sites have been underlined.
**Figure 5:** Elastic fibers in selected tissues of homozygous 3C7 mice. Sections were stained with Weigert's resorcin-fuchsin and analyzed at x 200 magnification. (+/+ wild type mice; /- 3C7 mice).
**Figure 6:** Intracellular versus extracellular TGF β 1 in selected mouse tissues. Tissue sections were stained with polyclonal TGF- β antibodies specific for the intracellular (LC) or extracellular (CC) form of TGF- β 1.

### EXAMPLES

The invention is exemplified by the following illustrative but non-limiting examples:

### Example 1. A stem cell clone recovered from a gene trap screen generates a complex disease phenotype in transgenic mice

From a larger screen for gene trap integrations into genes induced during ES cell differentiation (Thorey, I.S. *et al.* (1998) *Mol Cell Biol* 18, 3081-3088), one integration was obtained (designated herein as 3C7) that generated a complex disease phenotype when bred to homozygosity in transgenic mice.

129/Sv strain derived (D3) ES cells were grown on irradiated (32 Gy) MEF feeder layers in DMEM supplemented with 15% (v/v) preselected and heat inactivated fetal calf serum (FCS) (Linaris, Bettingen, Germany), 100 mM nonessential amino acids (Gibco), 0.1 mM mercaptoethanol (Sigma), 1000 U/ml of leukemia inhibitory factor (LIF) (Esgro^{R}; Gibco /BRL), and 5 mg/ml penicillin and streptomycin. Differentiation was induced by withdrawing LIF and feeder layers for 4 days (Thorey, I.S. *et al*. (1998) *Mol Cell Biol* **18,** 3081-3088). Infection of ES cells with the U3Cre gene trap virus was performed by incubating for 2 hours 150 ES cells with 50 µl of virus containing supernatant described previously (Thorey, I.S. *et al.* (1998) *Mol Cell Biol* 18, 3081-3088).

129/Sv (D3) ES cell derived chimeras were generated by injecting C57BU6 blastocysts. The resulting male chimeras were bred to C57BU6 females and agouti offspring were tested for transgene transmission by tail blotting. Mouse tail DNA was cleaved with BgIII which does not cut provirus. The DNA was fractionated on 1% agarose gels, blotted onto Hybond N nylon filters (Amersham/ Pharmacia, Piscataway, NJ) and hybridized to a ³²P labeled provirus flanking sequence probe. Animals heterozygous for the gene trap insertion were backcrossed to C57BU6 mice for at least six generations before analyzing the phenotypes in heterozygous and homozygous offspring.

By the age of four weeks, 3C7 mice developed a rectal prolapse induced by an extensive edema in the mucosal layer of the colorectal region (Figure 1 A, left panel). Paraffin sections of mouse tissues were prepared and stained according to standard histopathology procedures known to the person skilled in the art. Microscopically, the region exhibited dysplastic and/or collapsed crypts with an increased number of goblet cells (data not shown). Lesions of this type are consistent with a preneoplastic stage of colon cancer and, indeed, by the age of 4-6 months, 3C7 mice developed visible tumors with diameters of 0.7 - 1.5 cm (Figure 1A, right panel). Histologically, the tumors consisted of irregular crypts formed by highly proliferative anaplastic cells that infiltrated the mucosal and smooth muscle layers of the colonic wall (Figure 1 B). This pathology is typical for aggressive adenocarcinomas and is similar to the carcinomas developing in Smad 3 knock-out mice (Zhu, Y., et al. (1998) *Cell* 94, 703-714). As Smad 3 is a downstream target of TGF- β, the similarity between the tumors suggested that the tumors in both Smad 3 knock out- and 3C7 mice developed as a consequence of defective TGF-β signaling.

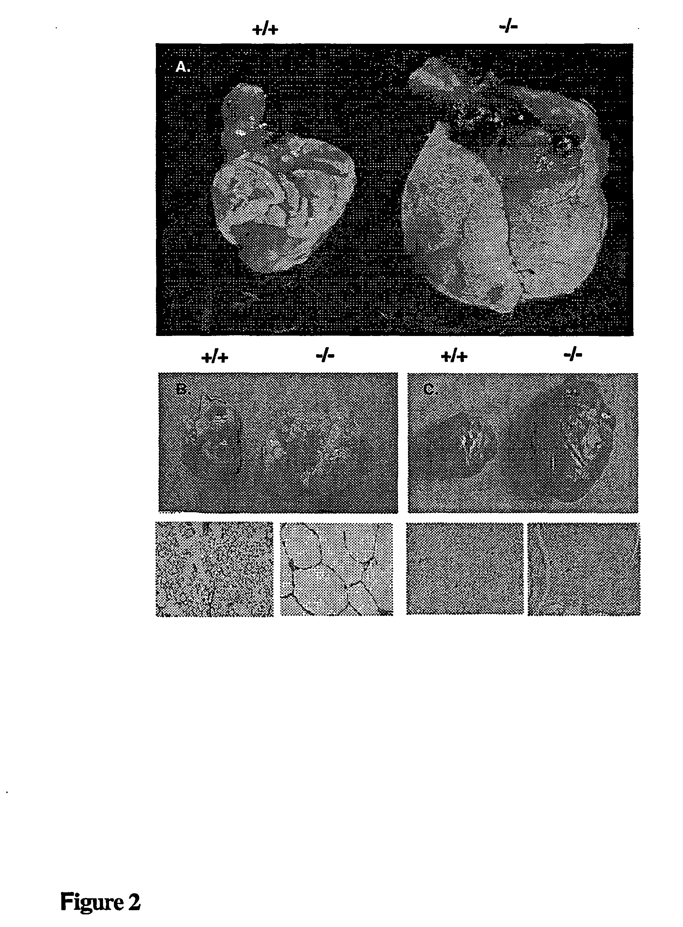

In addition to the tumors, 3C7 mice developed a pulmonary emphysema which, unlike the tumors, was already present at birth. The emphysema worsened with time such that by age of 4 - 6 months the lungs of 3C7 mice exceeded normal size by 4 fold (Figure 2 A, B). Histologically, alveolar spaces were enlarged, inflated and significantly reduced in number. They were surrounded by thin, dysplastic and frequently incomplete septal walls (Figure 2 B). The lobular connective tissue was diminished and replaced by mutifocal atelectatic areas. This resulted in an almost complete loss of pulmonary elasticity. The emphysema was associated with a cardiomegaly that involved biventricular dilation and myofibrillar hypertrophy (Figure 2 C). In most cases the cardiac/body weight index exceeded the normal value by 30%.

Clinically, animals were healthy up to 4-6 months of age but thereafter their general condition deteriorated. At this stage, mice were sacrificed due to severe dyspnea and prolapse infections that frequently progressed towards ulcerative proctitis.

Autopsy revealed a massive inflammatory infiltration of the colonic wall that was associated with reactive splenomegaly¹.
¹ All the other organs and tissues were microscopically normal. Analysis included liver, kidney, intestine, lymph nodes, brain, bone marrow, thymus, adrenals, pituitary, thyroid, pancreas, ovaries, uterus and testicles.

As heterozygous mice were indistinguishable from wild type mice and transgene inheritance was consistent with a Mendelian pattern, it was concluded that the gene trap had disrupted the function of an autosomal recessive gene.

### Example 2. The gene trap integration in 3C7 mice is located in the 5^{th} intron of the LTBP-4 gene

To identify the gene responsible for the 3C7 phenotype, 5'RACE was used to isolate cellular sequence co-transcribed with the provirus (gene trap sequence tag, GTST) (Wiles, M.V. *et al*. (2000) *Nat Genet* 24, 13-14). Normally, the type of retroviral gene trap employed in these experiments selects for integrations into exons (exon trap) and in most cases sequences upstream of the integration site are transcribed as cell-provirus fusion transcripts which can be easily identified by RT-PCR (Wempe, F. et al. (2001) *Genome Biol* 2, research0023.1-0023.10). Since 5'RACE and Northern blotting showed no cell provirus fusion transcripts (Figure 3B) it was concluded that in this case the gene trap, which is normally transcribed from an upstream cellular promoter, was activated instead by a nearby cellular enhancer. Enhancer activations with this type of gene trap have been observed, as it still contains a minimal promoter in the 5'LTR (Figure 3B) (Thorey, I.S. *et al*. (1998) *Mol Cell Biol* 18, 3081-3088). Taken together, these results showed that the gene trap had integrated into an intron and was therefore inaccessible to cloning by RT-PCR. Inverse PCR and genome walking was used to isolate the genomic sequence spanning the provirus integration site.

Inverse PCR from genomic DNAs was performed using the blunt end restriction enzyme Sspl and Cre-specific primers (Wempe, F., et al. (2001) *Genome Biol* **2**, research0023. 1-0023.10). A 210 nt amplification product was cloned into the p-GEMT-vector (Promega, Madison, WI) and sequenced using an ABI 310 Genetic Analyzer (Perkin-Elmer, Foster, CA). The upstream provirus flanking sequence was extended by genome walking using the mouse GenomeWalker kit (Clontech, Palo Alto, CA) according to the manufacturer's instructions and the specific flanking sequence forward primers 5'-AAGAGGGAGAAGAAACTGGGCTGG-3' (SEQ ID NO:3) and 5'-TCCTGTAGTGTTTGCCCCTG-3' (SEQ ID NO:4). A 781 bp genomic contig spanning the proviral integration site was amplified from the kit's EcoRV library and used as a probe to screen the lambda-TripleEx 5«-Stretch PLUS mouse kidney cDNA library (Clontech, Palo Alto, CA) and the lambda EMBL3 mouse liver genomic library (Clontech, Palo Alto, CA). Positive plaques were purified by standard methodology and inserts were sequenced after subcloning into Bluescript vectors.

The 781 nt contig was cloned and sequenced and used to find matches in the public data bases. Cellular sequences adjacent to proviral integrations (gene trap sequence tags; GTSTs) were searched in the NCBI/NIH genomic databases (http://www.ncbi.nlm.nih) using the BlastN algorithm. As no matches were found after extensive searching, it was concluded that the trapped gene is still unknown. To identify the gene, cDNA- and genomic libraries were screened with the genomic contig with methods known to the person skilled in the art, thus resulting in the isolation of a 12 kb genomic fragment with two short stretches of homology to the human cDNA encoding the latent transforming growth factor beta binding protein 4 (LTBP-4; Genbank Acc.# AF051344; ref|NM/003573.1; AF051345). The matching segments corresponded to exons 6 and 7 encoding the 5'end of the first EGF-repeat, which is shared among known splice variants (Saharinen, J. et al. (1998) *J Biol Chem* 273, 18459-18469). (Figure 3A). The splice variants LTBP-4L (long splice variant) and LTBP-4S (short splice variant) of the mouse LTBP-4 cDNA are shown in SEQ ID NO:1 and SEQ ID NO:2, respectively.

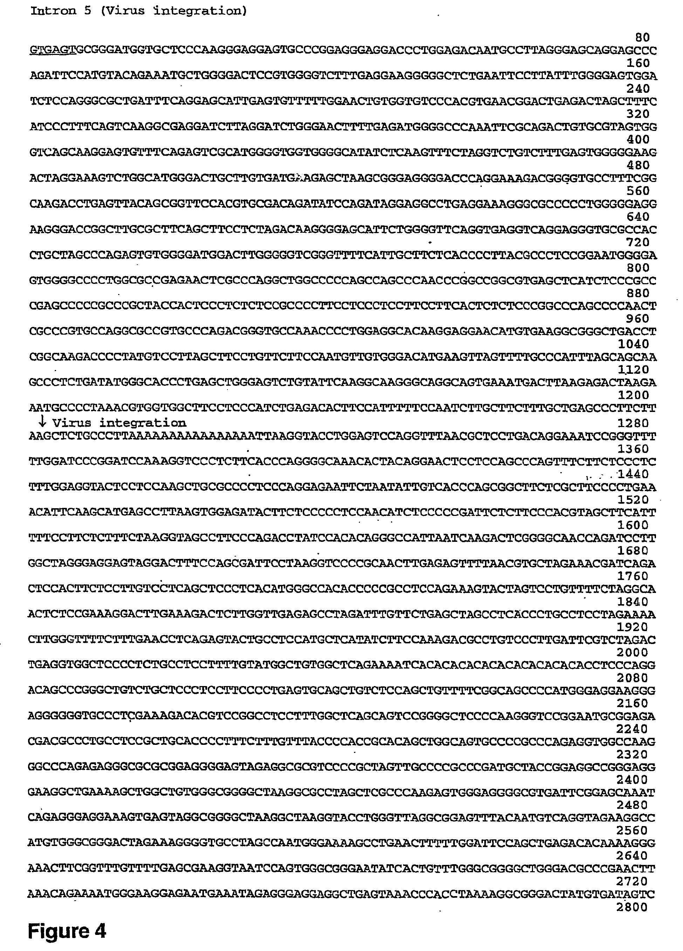

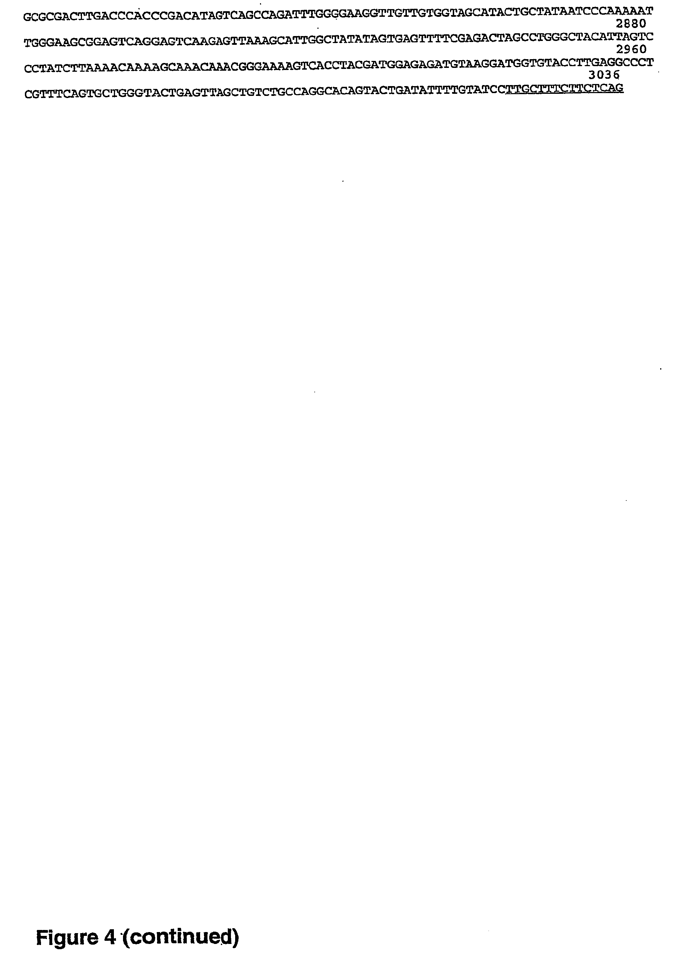

Figure 3A shows that the gene trap integration had occurred into the 5^{th} intron of the mouse LTBP-4 gene in a reverse transcriptional orientation relative to the gene. This inverted orientation explains the absence of a cell-provirus fusion transcript and supports a gene trap activation by a cellular enhancer. The sequence of intron 5 of the mouse LTBP-4 which contains the gene trap integration is shown in Figure 4 and SEQ ID NO:5.

### Example 3. The proviral integration in LTBP4 severely interrupts gene expression

To investigate whether the proviral intron disruption interfered with gene expression, mRNA from a variety of mouse tissues was hybridized on Northern blots to an LTBP-4 specific probe. For Northern blots 2.5 µg of poly(A)⁺ mRNAs purified with the FastTrack 2.0 mRNA purification kit (Invitrogen, Carlsbad, CA) were fractionated in 1% formaldehyde-agarose gels, transferred onto Hybond N nylon filters and hybridized to a ³²P-dCTP-labeled LTBP-4 specific probe. The probe was a 505 bp fragment located between nucleotides 862 - 1367 in the mouse LTBP-4S cDNA (SEQ ID NO:2). All probes were prepared by random priming using the Rediprime (Amersham / Pharmacia, Piscataway, US) kit. Blots were exposed to Kodak-Biomax autoradiography film. As shown in Figure 3 C (top), mice homozygous for the gene trap integration expressed less than 2% of normal LTBP-4 mRNA. Transcript levels in wild type mice were significantly higher in the lung, heart and colon than in other tissues, demonstrating a link between the phenotype and LTBP-4 expression (Figure 3 C).

To test whether protein levels were similarly decreased, the same range of tissues was analyzed by immunoblotting using an anti-LTBP-4 specific antibody (Saharinen, J., et al. (1998) *J Biol Chem* 273, 18459-18469). Equal amounts of selected tissues were homogenized in ice-cold RIPA buffer (150 mM NaCl, 1% NP-40, 0.5% sodium deoxycholate in 50 mM Tris-HCl, pH 7.2). Following centrifugation, the deoxycholate insoluble pellet was washed in PBS and digested with plasmin (Taipale et al., (1992) *J. Biol. Chem.* 267:25378-25384). After adding protease inhibitors (Roche Diagnostics, Mannheim, Germany), the supernatants were concentrated in Microcon 30 concentrators (Amicon, Millipore Corp., Bedford, MA). Proteins were fractionated on 7% SDS/PAGE gels and transferred to Protran nitrocellulose membranes (Schleicher & Schuell, Dassel, Germany). Immunodetection after treatment with polyclonal anti-LTBP-1 or anti-LTBP-4 antibodies was performed (Saharinen et al. (1998) *J Biol Chem* 273, 18459-18469). As shown in Figure 3 C (bottom), protein was hardly detectable in 3C7 mice and levels closely followed the pattern of mRNA expression in the wild type mice. Taken together, the results showed that the gene trap integration into the 5^{th} intron of the LTBP-4 gene resulted in a nearly null allele.

### Example 4. Elastic fibers in the lung and colon require LTBP-4

Together with fibrillins, LTBPs form a sheath of microfibrills that surrounds the elastic fiber's elastin core. Fibrillins are essential for the integrity of elastic fibers and mutations in the fibrillin genes are common in connective tissue disorders such as Marfan's syndrome. Since it was shown that 3C7 mice fail to assemble elastic fibers, particularly in the lung and colon, the animal of the present invention is a suitable model to investigate the role of LTBP-4 for the assembly of elastic fibers. To test this, elastic fibers were visualized using an elastin-specific histological stain. Tissues were fixed in 4 % buffered formalin, processed through a graded series of alcohol, cleared in xylene and placed in a tissue processor for 8 hours. 5 µm (micron) paraffin sections were prepared with a rotary microtome, placed on lysine coated glass slides. After incubating for 45 minutes at 65°C, slides were deparaffinized in xylene. After rehydrating in double distilled H₂O, slides were immersed in Weigert's resorcin-fuchsin solution (70% v/v ethanol, 1% v/v hydrochloric acid conc, and 0.2% v/v resorcin-fuchsin [Chroma, Münster, Germany]) for 6 hours at room temperature. Following washing in ddH₂O slides were dehydrated in alcohol and mounted in permount.

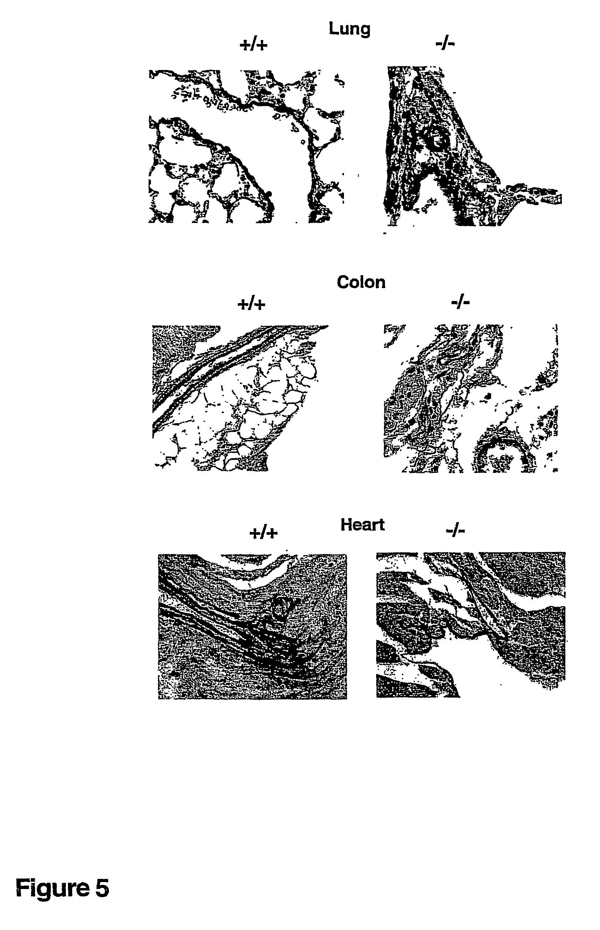

Figure 5 shows that the lung and colon of 3C7 mice lacked elastic fibers and exhibited instead multiple patches of fragmented and condensed elastin (Figure 5). Surprisingly, elastic fibers in the heart appeared unaltered by the mutation, indicating that the severe cardiomyopathy is at least in part secondary to the emphysema (Figure 5). Taken together, the results show that LTBP-4 is essential for the assembly of elastic fibers.

### Example 5. Export of TGF- β 1 to the ECM requires LTBP-4

It has recently been shown that of all TGF- β isoforms, only TGF β 1 binds LTBP-4 in vitro (Saharinen, J. & Keski-Oja, J. (2000) *Mol Biol Cell* 11, 2691-2704). Furthermore, LTBPs are responsible for the export of TGF- β to the ECM. To test if mice lacking LTBP-4 would also lack extracellular TGF β 1, anti-TGF- β 1 antibodies were used that recognize TGF- β 1 epitopes selectively displayed inside (LC) or outside (CC) the cell (Flanders, K.C. *et al.* (1989) *J Cell Biol* 108, 653-660, Thompson, N.L. *et al.* (1989) *J Cell Biol* 108, 661-669). For immunostainings, deparaffinized and rehydrated tissue slides were first treated for 30 minutes with 30% H₂O₂ to block the endogenous peroxidase. After rinsing in ddH₂O and soaking in PBS for 5 - 10 minutes, slides were treated with 10 % w/v BSA in PBS to eliminate nonspecific protein binding sites. The slides were then exposed ovemight at 4 °C to the polyclonal TGF-β1 anti-CC(1-30) and anti-LC(1-30) antibodies at concentrations of 1 µg/ml and 4 µg/ml, respectively (Flanders, K.C. et al. (1989) *J Cell Biol* **108,** 653-660). After removing excess antibody, slides were treated with biotin-labeled anti-mouse (Dianova, Hamburg) antibody for 30 minutes at 37 °C and finally with horse-radish peroxidase (HRP) labeled streptavidine (Zymed, CA) for 20 minutes at 37°C. After washing, slides were incubated in diaminobenzidine (Sigma, St. Louis, MO) for 10 minutes at room temperature. Slides were examined under a conventional microsope after removing the excess substrate in ddH₂O.

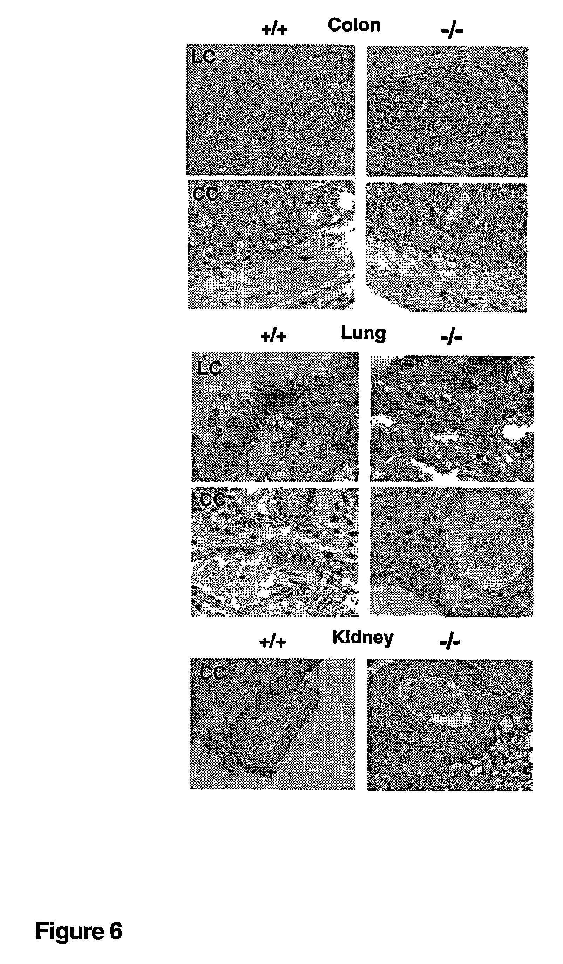

Immunohistochemical analysis showed that in 3C7 mice the lung, colon and heart contained no extracellular TGF-β 1. As intracellular levels were normal, the results demonstrate a defect in exporting TGF-β 1 to the ECM. This defect is tissue specific since the kidneys of 3C7 mice had normal extracellular TGF-β 1 levels (Figure 6).

## Claims

1. A non-human animal model which does not produce functional latent transforming growth factor β binding protein 4 (LTBP-4) or which produces suboptimal levels of latent transforming growth factor β binding protein 4 (LTBP-4), wherein said animal exhibits cancer, pulmonary emphysema or cardiomyopathy.

2. The animal model of claim 1, wherein the genome of the animal comprises a homozygous disruption of the LTBP-4 gene.

3. The animal model of claim 2, wherein said homozygous disruption has been generated by a mutation.

4. The animal model of claim 3, wherein said mutation is an insertion, deletion or a substitution mutation.

5. The animal model of claim 3, wherein said mutation is generated by gene targeting, gene trap integration or mutagenesis.

6. The animal model of claim 3, wherein the mutation has occurred in an exon, intron, regulatory region or splicing site of the LTBP-4 gene.

7. The animal model of claim 6, wherein the mutation has occurred in intron 5 of the LTBP-4 gene.

8. The animal model of claim 7, wherein the cancer is a colon cancer.

9. The animal model of claim 1, wherein said animal is a mammal.

10. The animal mode! of claim 9, wherein said mammal is a mouse or a rat.

11. A cell isolated from a non-human animal model whose genome comprises a homozygous disruption of the LTBP-4 gene such that said gene does not produce functional latent transforming growth factor β binding protein 4 or such that said gene produces suboptimal levels of latent transforming growth factor β binding protein 4.

12. The cell of claim 11, wherein the cell is from a colon, lung or heart.

13. A non-human cell whose genome comprises a homozygous disruption of the LTBP-4 gene.

14. Tne cell of claim 13, wherein the cell is an embryonic stem cell.

15. A method for selecting an agent for treating a symptom occurring in the animal model of claim 1 comprising:
a) applying one or more agents to be tested to the animal model of claim 1,
b) determining whether one or more symptoms occurring in the animal model of claim 1 have changed as a result of application of said agent or agents.

16. The method of claim 15, wherein the symptom is selected from a group consisting of cancer, pulmonary emphysema and cardiomyopathy.

17. A method to analyze whether cancer and/or pulmonary emphysema and/or cardiomyopathy is caused by differential LTBP-4 gene or protein expression or expression level or by a defect in the LTBP-4 gene comprising:
a) characterizing the LTBP-4 gene or protein expression or expression level or LTBP-4 gene allele status of an individual having cancer, pulmonary emphysema or cardiomyopathy,
b) characterizing the LTBP-4 gene or protein expression or expression level or LTBP-4 gene allele status of a control,
wherein the method is not practised on the human or animal body and wherein a difference in the LTBP-4 gene or protein expression or expression level or LTBP-4 gene allele status indicates that cancer and/or pulmonary emphysema and/or cardiomyopathy is caused by differential LTBP-4 gene or protein expression or expression level or a defect in the LTBP-4 gene.

18. A method for diagnosing cancer and/or pulmonary emphysema and/or cardiomyopathy comprising:
a) characterizing the LTBP-4 gene or protein expression or expression level or LTBP-4 gene allele status of an individual,
b) characterizing the LTBP-4 gene or protein expression or expression level or LTBP-4 gene allele status of a control,
wherein the method is not practised on the human or animal body and wherein a difference in the LTBP-4 gene or protein expression or expression level or LTBP-4 gene allele status indicates the presence of cancer and/or pulmonary emphysema and/or cardiomyopathy in said individual.

19. The method of claim 17 or 18, wherein the LTEP-4 expression or expression level is detected by RT-PCR, Northern analysis, microarray analysis or antibodies directed to LTBP-4 protein.

20. The method of claim 17 or 18 wherein the LTBP-4 gene allele status is detected by mutation screening.

## Patentansprüche

1. Nichthumanes Tiermodell, das kein funktionelles latentes Transformierender-Wachstumsfaktor-β-Bindungsprotein 4 (LTBP-4) produziert oder das suboptimale Mengen an latentem Transformierender-Wachstumsfaktor-β-Bindungsprotein 4 (LTBP-4) produziert, wobei das Tier Krebs, Lungenemphysem oder Kardiomyopathie aufweist.

2. Tiermodell gemäß Anspruch 1, wobei das Genom des Tiers eine homozygote Zerstörung des LTBP-4-Gens umfasst.

3. Tiermodell gemäß Anspruch 2, wobei die homozygote Zerstörung durch eine Mutation entstanden ist.

4. Tiermodell gemäß Anspruch 3, wobei die Mutation eine Insertions-, Deletions- oder Substitutionsmutation ist.

5. Tiermodell gemäß Anspruch 3, wobei die Mutation durch Gen-Targeting, Genfallenintegration oder Mutagenese entstanden ist.

6. Tiermodell gemäß Anspruch 3, wobei die Mutation in einem Exon, Intron, regulatorischen Bereich oder einer Spleißstelle des LTBP-4-Gens aufgetreten ist.

7. Tiermodell gemäß Anspruch 6, wobei die Mutation in Intron 5 des LTBP-4-Gens aufgetreten ist.

8. Tiermodell gemäß Anspruch 7, wobei der Krebs ein Kolonkarzinom ist.

9. Tiermodell gemäß Anspruch 1, wobei das Tier ein Säuger ist.

10. Tiermodell gemäß Anspruch 9, wobei der Säuger eine Maus oder eine Ratte ist.

11. Zelle, die aus einem nichthumanen Tiermodell isoliert wurde, dessen Genom eine homozygote Zerstörung des LTBP-4-Gen umfasst, so dass dieses Gen kein funktionelles latentes Transformierender-Wachstumsfaktor-β-Bindungsprotein 4 (LTBP-4) produziert oder suboptimale Mengen an latentem Transformierender-Wachstumsfaktor-β-Bindungsprotein 4 (LTBP-4) produziert.

12. Zelle gemäß Anspruch 11, wobei die Zelle aus einem Kolon, einer Lunge oder einem Herzen stammt.

13. Nichthumane Zelle, deren Genom eine homozygote Zerstörung des LTBP-4-Gen umfasst.

14. Zelle gemäß Anspruch 13, wobei die Zelle eine embryonale Stammzelle ist.

15. Verfahren zum Auswählen eines Mittels zur Behandlung eines Symptoms, das im Tiermodell von Anspruch 1 auftritt, umfassend:
a) Anwenden von einem oder mehreren zu testenden Mitteln auf das Tiermodell von Anspruch 1;
b) Bestimmen, ob ein oder mehrere Symptome, die in dem Tiermodell von Anspruch 1 auftreten, sich infolge der Anwendung des oder der Mittel verändert haben.

16. Verfahren gemäß Anspruch 15, wobei das Symptom aus einer Gruppe ausgewählt ist, die aus Krebs, Lungenemphysem und Kardiomyopathie besteht.

17. Verfahren zum Analysieren, ob Krebs und/oder Lungenemphysem und/oder Kardiomyopathie durch ein unterschiedliches LTBP-4-Gen oder unterschiedliche Proteinexpression oder ein unterschiedliches Expressionsniveau oder durch einen Defekt im LTBP-4-Gen verursacht wird, umfassend:
a) Charakterisieren des LTBP-4-Gens oder der Proteinexpression oder des Expressionsniveaus oder des LTBP-4-Gen-Allelstatus eines Individuums, das Krebs, Lungenemphysem oder Kardiomyopathie hat;
b) Charakterisieren des LTBP-4-Gens oder der Proteinexpression oder des Expressionsniveaus oder des LTBP-4-Gen-Allelstatus einer Kontrolle;
wobei das Verfahren nicht am menschlichen oder tierischen Körper praktiziert wird und wobei ein Unterschied im LTBP-4-Gen oder in der Proteinexpression oder im Expressionsniveau oder im LTBP-4-Gen-Allelstatus darauf hinweist, dass Krebs und/oder Lungenemphysem und/oder Kardiomyopathie durch ein unterschiedliches LTBP-4-Gen bzw. unterschiedliche Proteinexpression bzw. ein unterschiedliches Expressionsniveau bzw. durch einen Defekt im LTBP-4-Gen verursacht wird.

18. Verfahren zum Diagnostizieren von Krebs und/oder Lungenemphysem und/oder Kardiomyopathie, umfassend:
a) Charakterisieren des LTBP-4-Gens oder der Proteinexpression oder des Expressionsniveaus oder des LTBP-4-Gen-Allelstatus eines Individuums;
b) Charakterisieren des LTBP-4-Gens oder der Proteinexpression oder des Expressionsniveaus oder des LTBP-4-Gen-Allelstatus einer Kontrolle;
wobei das Verfahren nicht am menschlichen oder tierischen Körper praktiziert wird und wobei ein Unterschied im LTBP-4-Gen oder in der Proteinexpression oder im Expressionsniveau oder im LTBP-4-Gen-Allelstatus auf Krebs und/oder Lungenemphysem und/oder Kardiomyopathie bei dem Individuum hinweist.

19. Verfahren gemäß Anspruch 17 oder 18, wobei die LTBP-4-Expression oder das LTBP-4-Expressionsniveau durch RT-PCR, Northern-Analyse, Mikroarray-Analyse oder gegen LTBP-4-Protein gerichtete Antikörper nachgewiesen wird.

20. Verfahren gemäß Anspruch 17 oder 18, wobei der LTBP-4-Gen-Allelstatus durch Mutations-Screening nachgewiesen wird.

## Revendications

1. Modèle animal non-humain qui ne produit pas de protéine 4 liant le facteur de croissance β transformant latent (LTBP-4) fonctionnelle ou qui produit des niveaux sous-optimaux de protéine 4 liant le facteur de croissance β transformant latent (LTBP-4), tandis que ledit animal présente un cancer, un emphysème pulmonaire ou une cardiomyopathie.

2. Modèle animal selon la revendication 1, dans lequel le génome de l'animal comprend une rupture homozygote du gène LTBP-4.

3. Modèle animal selon la revendication 2, dans lequel ladite rupture homozygote a été provoquée par une mutation.

4. Modèle animal selon la revendication 3, dans lequel ladite mutation est une insertion, une délétion ou une mutation par substitution.

5. Modèle animal selon la revendication 3, dans lequel ladite mutation est générée par ciblage de gène, intégration d'un piège à gène ou mutagenèse.

6. Modèle animal selon la revendication 3, dans lequel la mutation est intervenue dans un exon, un intron, une région régulatrice ou un site d'épissage du gène LTBP-4.

7. Modèle animal selon la revendication 6, dans lequel la mutation est intervenue dans l'intron 5 du gène LTBP-4.

8. Modèle animal selon la revendication 7, dans lequel le cancer est un cancer du côlon.

9. Modèle animal selon la revendication 1, dans lequel ledit animal est un mammifère.

10. Modèle animal selon la revendication 9, dans lequel ledit mammifère est une souris ou un rat.

11. Cellule isolée d'un modèle animal non-humain dont le génome comprend une perturbation homozygote du gène LTBP-4 telle que ledit gène ne produit pas de protéine 4 liant le facteur de croissance transformant β latent fonctionnelle ou telle que ledit gène produit des niveaux sous-optimaux de protéine 4 liant le facteur de croissance transformant β latent.

12. Cellule selon la revendication 11, dans laquelle la cellule est issue d'un côlon, poumon ou coeur.

13. Cellule non-humaine, dont le génome comprend une perturbation homozygote du gène LTBP-4.

14. Cellule selon la revendication 13, dans laquelle la cellule est une cellule souche embryonnaire.

15. Procédé pour la sélection d'un agent pour le traitement d'un symptôme se manifestant dans le modèle animal selon la revendication 1, comprenant:
a) l'application d'un ou plusieurs agents à tester au modèle animal selon la revendication 1,
b) la détermination de ce qu'un ou plusieurs symptômes apparaissant dans le modèle animal selon la revendication 1 ont ou non été modifiés à la suite de l'application du dit agent ou des dits agents.

16. Procédé selon la revendication 15, dans lequel le symptôme est choisi dans un groupe consistant en cancer, emphysème pulmonaire et cardiomyopathie.

17. Procédé pour analyser si un cancer et/ou un emphysème pulmonaire et/ou une cardiomyopathie est provoqué par une expression différentielle ou un niveau d'expression différentielle du gène ou de la protéine LTBP-4 ou par un défaut dans le gène LTBP-4, comprenant:
a) la caractérisation de l'expression ou du niveau d'expression du gène ou de la protéine LTBP-4 ou de l'état allélique du gène LTBP-4 d'un individu ayant un cancer, un emphysème pulmonaire, ou une cardiomyopathie,
b) la caractérisation de l'expression ou du niveau d'expression du gène ou de la protéine LTBP-4 ou de l'état allélique du gène LTBP-4 d'un témoin,
tandis que le procédé n'est pas mis en oeuvre sur le corps humain ou animal, et
tandis qu'une différence dans l'expression ou le taux d'expression du gène ou de la protéine LTBP-4 ou dans l'état allélique du gène LTBP-4 indique qu'un cancer et/ou un emphysème pulmonaire et/ou une cardiomyopathie est provoqué par l'expression ou le niveau d'expression différentielle du gène ou de la protéine LTBP-4 ou de la protéine, ou un défaut dans le gène LTBP-4.

18. Procédé pour le diagnostic d'un cancer et/ou d'un emphysème pulmonaire et/ou d'une cardiomyopathie, comprenant:
a) la caractérisation de l'expression ou du niveau d'expression du gène ou de la protéine LTBP-4 ou de l'état allélique du gène LTBP-4 pour un individu,
b) la caractérisation de l'expression ou du niveau d'expression du gène ou de la protéine LTBP-4 ou de l'état allélique du gène LTBP-4 d'un témoin,
tandis que le procédé n'est pas mis en oeuvre sur le corps humain ou animal, et
tandis qu'une différence dans l'expression ou le niveau d'expression du gène ou de la protéine LTBP-4 ou dans l'état allélique du gène LTBP-4 indique la présence d'un cancer et/ou d'un emphysème pulmonaire et/ou d'une cardiomyopathie chez ledit individu.

19. Procédé selon la revendication 17 ou 18, dans lequel l'expression ou le niveau d'expression de LTBP-4 est détecté par RT-ACP, analyse de Northem, analyse sur micro-arrangement ou des anticorps dirigés contre la protéine LTBP-4.

20. Procédé selon la revendication 17 ou 18, dans lequel l'état allélique du gène LTBP-4 est détecté par criblage des mutations.
